# EUROPÄISCHE PATENTSCHRIFT

(11) **EP 0 881 275 B1**
(45) Veröffentlichungstag und Bekanntmachung des Hinweises auf die Patenterteilung: **22.01.2003**
(21) Anmeldenummer: 98108390.0
(22) Anmeldetag: 08.05.1998
(51) Int. Cl.: C10G 45/00

(54) **Verfahren zur katalytischen Gasphasenhydrierung von Olefinen**
Process for catalytic gasphase hydrogenation of olefins
Procédé d'hydrogénation d'oléfines en phase gazeuse

(30) Priorität: 12.05.1997 DE 19719833
(43) Veröffentlichungstag der Anmeldung: 02.12.1998
(73) Patentinhaber: BASF AKTIENGESELLSCHAFT, 67056 Ludwigshafen (DE)
(72) Erfinder: Vicari, Maximilian, 67177 Limburgerhof (DE); Walter, Marc, 67227 Frankenthal (DE); Dilling, Stephan, 69469 Weinheim (DE)
(74) Vertreter: Isenbruck, Günter, Dr.

(56) Entgegenhaltungen:
- WO-A-86/05801
- WO-A-97/29841
- BE-A- 880 595
- US-A- 4 124 650
- US-A- 4 203 828

## Beschreibung

Die Erfindung betrifft ein Verfahren zur Gasphasenhydrierung von Olefinen, wobei die Olefine nicht in einem Verdampfer vorverdampft werden.

Bei der Oligomerisierung von leichten Olefinen, beispielsweise Ethylen, Propylen und Butylen fallen C₆- bis C₂₀-Olefine an. Diese Olefine befinden sich hauptsächlich in Crackgasen, wie sie beispielsweise in FCC- oder SC-Anlagen entstehen, oder werden durch die Dehydrierung aus den entsprechenden Paraffinen erzeugt. Die Oligomerisierung dieser leichten Olefine kann in Gegenwart von einem homogenen oder einem heterogenen Katalysator durchgeführt werden. Die dabei anfallenden C₆- bis C₂₀-Olefine können zu Kraftstoffen weiterverarbeitet werden. Besonders Isooctan eignet sich hervorragend als Otto-Kraftstoff-Komponente, da sich diese Verbindung durch einen hohen RON-Wert und hohen MON-Wert auszeichnet.

N.L. Allinger et al., Organische Chemie, S. 481 ff, de Gruyter, 1980, offenbart, die Hydrierung von Doppelbindungen bei niedrigen Drücken (1 bis 4 bar) und bei mäßigen Temperaturen (< 100 °C) an Edelmetallen der VIII. Nebengruppe des Periodensystems (Platin, Palladium und auch Rhodium). Weiterhin ist diesem Stand der Technik auch die Hydrierung von Doppelbindungen mit Nickelkatalysatoren zu entnehmen.

Bisher war es bei schonenden Gasphasenhydrierverfahren notwendig, die olefinhaltige Zugabe vor der Einspeisung in das Gas des Reaktionssystems, insbesondere im Reaktor, einem Verdampfer zuzuführen, indem die Zugabe von der flüssigen in die gasförmige Phase für die Gasphasenhydrierung gebracht wird. Im allgemeinen handelt es sich bei derartigen Verdampfer um beheizte Bereiche, die vorzugsweise vor dem Reaktor angeordnet sind und durch die das Hydriergas strömt. Diese Vorverdampfung ist mit einem hohen apparativen und Energieaufwand verbunden und stellt demgemäß insbesondere einen wirtschaftlichen Nachteil dar.

Aus der belgischen Patentschrift 880.595 ist ein Verfahren zur Herstellung von paraffinischen Lösungsmitteln durch Gasphasenhydrierung von Oligomeren bekannt, in dem jedoch die Gasphasenhydrierung nicht mit der erfindungsgemäßen Form der Einspeisung erfolgt.

Der Erfindung liegt somit das Problem zugrunde, ein Verfahren zur Gasphasenhydrierung von olefinhaltigen Zugaben zur Verfügung zu stellen, bei dem die Reaktionsbedingungen, insbesondere Temperatur, Druck, Kohlenwasserstoffbelastung, das Verhältnis zwischen Gas und Zugabe, und die Reaktionsführung, insbesondere die Art der Einspeisung, so ausgewählt sind, daß die Gasphasenhydrierung ohne Vorverdampfung der Zugabe mit hohen Ausbeuten durchgeführt werden kann.

Dieses wurde überraschend durch ein Verfahren zur Gasphasenhydrierung von einer mindestens 50 Gew.-% mindestens eines C₆- bis C₂₀-Olefins beinhaltenden Zugabe mit einem mindestens Wasserstoff beinhaltenden Gas an einem Katalysator, wobei die Zugabe als Flüssigkeit in das Gas eingespeist wird, gelöst.

Vorzugsweise erfolgt das zuvor genannte erfindungsgemäße Verfahren zur Gasphasenhydrierung an einem mindestens ein Element der VIII. Nebengruppe des Periodensystems beinhaltenden Katalysator in einem Reaktionssystem, wobei mit mindestens einem, bevorzugt zwei und besonders bevorzugt allen Parametern, wie einem molaren Gas-Zugabe-Verhältnis im Bereich von 5 bis 200, in einem Gastemperaturbereich von 50 bis 300°C, einem Gasdruckbereich von 1 bis 20 bar, einer Katalysatorbelastung im Bereich von 0,25 bis 3 kg/l_{Katalysator}·h hydriert, das Gas in einem Gaskreislauf geführt wird.

### I. OLEFINZUGABE

Erfindungsgemäß beinhaltet die Zugabe alle dem Fachmann bekannten C₆bis C₂₀-Olefine. Unter den vorstehenden Olefinbegriff fallen Olefine mit einer oder mehreren Doppelbindungen sowie verzweigte und unverzweigte oder auch cyclische Olefine. Die erfindungsgemäß eingesetzte Zugabe beinhaltet mindestens 50, bevorzugt mindestens 75 und besonders bevorzugt mindestens 90 Gew.-% der vorgenannten oder auch nachstehend aufgeführten Olefine.

Eine erfindungsgemäß bevorzugte Zugabe beinhaltet im allgemeinen C₆- bis C₂₀-, vorzugsweise C₆- bis C₁₆-, bevorzugt C₆- bis C₁₃ - und besonders bevorzugt C₇- bis C₈- oder C₁₁- bis C₁₃- Olefine mit 1 bis 3, bevorzugt 1 bis 2 und besonders bevorzugt einer Doppelbindung, sowie deren Gemische.

Eine weiterhin erfindungsgemäß bevorzugte Zugabe beinhaltet vorzugsweise verzweigte C₆- bis C₂₀-, vorzugsweise C₆- bis C₁₆-, bevorzugt C₆- bis C₁₃und besonders bevorzugt C₇- bis C₈- oder C₁₁- bis C₁₃-Olefine mit 3, vorzugsweise 2 und besonders bevorzugt einer Doppelbindung.

Eine weiterhin erfindungsgemäß bevorzugte Zugabe beinhaltet 60 bis 80 Gew.-%, bezogen auf die gesamte Zugabe, ein verzweigtes C₆- bis C₂₀-, vorzugsweise C₆- bis C₁₆-, bevorzugt C₆- bis C₁₃- und besonders bevorzugt C₇- bis C₉-α-Olefin mit 1 bis 3, bevorzugt 1 bis 2 und besonders bevorzugt einer Doppelbindung.

Ferner beinhaltet eine erfindungsgemäße bevorzugte Zugabe 40 bis 20 Gew.-% bezogen auf die gesamte Zugabe, ein verzweigtes C₆- bis C₂₀-, vorzugsweise C₆- bis C₁₆-, bevorzugt C₆- bis C₁₃- und besonders bevorzugt C₇- bis C₉-β-Olefin mit 1 bis 3, bevorzugt 1 bis 2 und besonders bevorzugt einer Doppelbindung.

Eine erfindungsgemäß besonders bevorzugte Zugabe beinhaltet sowohl das verzweigte α-Olefin als auch das verzweigte β-Olefin der beiden vorhergehenden Abschnitte.

Darüber hinaus bevorzugt beinhaltet erfindungsgemäß eine Zugabe 60 bis 80 Gew.-%, bezogen auf die gesamte Zugabe, ein C₈-α-Olefin mit einer Doppelbindung, wobei es besonders bevorzugt ist, wenn das zuvor genannte Olefin verzweigt ist. Ebenso erfindungsgemäß bevorzugt ist eine Zugabe, die 40 bis 20 Gew.-%, bezogen auf die gesamte Zugabe, ein C₈-β-Olefin mit einer Doppelbindung beinhaltet, wobei es besonders bevorzugt ist, wenn dieses β-Olefin verzweigt ist. Eine über diese beiden erfindungsgemäßen Zugaben hinaus bevorzugte Zugabe beinhaltet sowohl das zuvor genannte C₈--α-Olefin als auch das zuvor genannte C₈-β-Olefin, jeweils mit einer Doppelbindung, besonders bevorzugt in den beiden zuletzt angegebenen Gew.-%-Bereichen, jeweils bezogen auf die gesamte Zugabe.

Eine darüber hinaus erfindungsgemäß bevorzugte Zugabe beinhaltet 60 bis 80, bevorzugt 65 bis 75 Gew.-%, bezogen auf die gesamte Zugabe, ein C₈-α-Olefin mit einer C₅-Hauptkette und einer Doppelbindung, besonders bevorzugt 2,4,4-Trimethyl-1-Penten. Eine ebenso erfindungsgemäß bevorzugte Zugabe beinhaltet 40 bis 20, bevorzugt 35 bis 25 Gew.-%, bezogen auf die gesamte Zugabe, C₈-β-Olefin mit einer C₅-Hauptkette und einer Doppelbindung, besonders bevorzugt 2,4,4-Trimethyl-2-Penten. Über diese in diesem Abschnitt genannten erfindungsgemäßen Zugaben ist eine Zugabe bevorzugt, die sowohl das C₈-α-Olefin mit einer C₅-Hauptkette und einer Doppelbindung als auch C₈-β-Olefin mit einer C₅-Hauptkette und einer Doppelbindung und besonders bevorzugt 2,4,4-Trimethyl-1-Penten und 2,4,4-Trimethyl-2-penten in den eingangs dieses Abschnitts aufgeführten Gew.-%-Bereichen beinhaltet.

Eine weitere erfindungsgemäße Zugabe beinhaltet mindestens 90% eines C₈oder C₁₂-Olefin-Schnittes.

Darüber hinaus bevorzugt sind Zugaben, die den C₆- bis C₂₀-, vorzugsweise C₆- bis C₁₆-Olefinfraktionen entsprechen, wie sie bei der Oligomerisierung von leichten Olefinen, beispielsweise Ethylen, Propylen und Butylen, als Haupt- oder Nebenprodukte anfallen. Besonders bevorzugt sind C₆- bis C₂₀-, vorzugsweise C₆- bis C₁₆-Olefinfraktionen, wie sie bei der großtechnischen Oligomerisierung von leichten Olefinen, beispielsweise Ethylen, Propylen und Butylen als Haupt- oder Nebenprodukte, insbesondere bei den großtechnischen Verfahren wie den Polygas-Verfahren von UOP Inc., dem Octolverfahren der Hüls AG sowie den Dimersolverfahren von IFP anfallen.

### II. GAS

Als Gas für das erfindungsgemäße Verfahren zur Gasphasenhydrierung von Olefinen kommt sowohl vorzugsweise ausschließlich Wasserstoff als auch Wasserstoff in Mischung mit Inertgasen in Betracht. Erfindungsgemäß bevorzugte Inertgase sind CH₄, Stickstoff und Edelgase, bevorzugt Stickstoff und Argon und besonders bevorzugt Stickstoff, darüber hinaus bevorzugt CH₄ oder deren Mischungen. Für das erfindungsgemäße Hydrierverfahren muß das Gas mindestens soviel Wasserstoff enthalten, wie es nach dem Grad der Hydrierung aufgrund der Anzahl der Doppelbindungen stöchiometrisch notwendig ist. Für den Fall, daß alle Doppelbindungen einer Zugabe hydriert werden sollen, ist es demnach notwendig, daß mindestens die der Anzahl der Doppelbindungen in der Zugabe entsprechende stöchiometrische Menge an Wasserstoff im Gas vorhanden ist. Die erfindungsgemäß im Gas bevorzugten Verhältnisse zwischen Wasserstoff und Inertgas ergeben sich aus Tabelle 1, wobei die Gew.-%-Angaben auf das gesamte Gas bezogen sind.

**Tabelle 1**

| **Gas** | **Gew.-%** | **bevorzugt Gew.-%** | **besonders bevorzugt Gew.-%** |
|---|---|---|---|
| H₂ -Gas | 50 bis < 100 | 80 bis < 100 | 90 bis < 100 |
| Inertgas | > 0 bis 50 | > 0 bis 20 | > 0 bis 10 |

### III. KATALYSATOR

Für das erfindungsgemäße Verfahren zur Gasphasenhydrierung von Olefinen sind die dem Fachmann geläufigen Katalysatoren, die mindestens ein Element der VIII. Nebengruppe enthalten, geeignet. Im erfindungsgemäßen Verfahren zur Gasphasenhydrierung können die vorzugsweise heterogenen Katalysatoren sowohl geträgert als auch bevorzugt als Vollkatalysator eingesetzt werden.

Erfindungsgemäß ist es bevorzugt, wenn in dem Katalysator neben mindestens einem Element der VIII. Nebengruppe noch mindestens ein Element ausgewählt aus der Gruppe der Elemente der III. und IV. Hauptgruppe sowie der IV. Nebengruppe vorliegt. Darüber hinaus ist es bevorzugt, wenn in dem Katalysator neben mindestens einem Element der VIII. Nebengruppe jeweils mindestens ein Element der III. und IV. Hauptgruppe sowie der IV. Nebengruppe vorliegt. Besonders bevorzugt im erfindungsgemäßen Verfahren eingesetzte Katalysatoren ergeben sich aus der nachfolgenden Tabelle 2, wobei die Gew.-%-Angaben auf den gesamten Katalysator bezogen sind.

**Tabelle 2**

| **Elemente, vorzugsweise deren Oxide** | **bevorzugt von [Gew.-%]*** | **besonders bevorzugt von [Gew.-%]*** |
|---|---|---|
| VIII. Nebengruppe, vorzugsweise Nickel | 55 bis 100 | 60 bis 97 |
| IV. Hauptgruppe, vorzugsweise Silizium | 0 bis 25 | 1 bis 20 |
| III. Hauptgruppe, vorzugsweise Aluminium | 0 bis 10 | 1 bis 10 |
| IV. Nebengruppe, vorzugsweise Zirkon | 0 bis 10 | 1 bis 10 |

| | | |
|---|---|---|
| * bezogen auf Nickeloxid, Siliziumoxid, Aluminiumoxid, Zirkonoxid | | |

Ein erfindungsgemäß besonders bevorzugter Katalysator beinhaltet 65 bis 80 Gew.-% Nickel, berechnet als Nickeloxid, 10 bis 25 Gew.-% Silizium, berechnet als Siliziumoxid, 2 bis 10 Gew.-% Zirkon, berechnet als Zirkonoxid, 0 bis 10 Gew.-% Aluminium, berechnet als Aluminiumoxid, vorzugsweise mit der Maßgabe, daß die Summe aus dem Gehalt an Siliziumdioxid und Aluminiumoxid mindestens 15 Gew.-%, bezogen auf die Gesamtmasse des Katalysators, beträgt, vorzugsweise erhältlich durch Zugabe einer sauren, wäßrigen Lösung von Nickel-, Zirkon- und gegebenenfalls Aluminiumsalzen zu einer basischen, wäßrigen Lösung unter Suspension von Siliziumverbindungen und gegebenenfalls Aluminiumverbindungen, wobei vorzugsweise der pH-Wert der so erhaltenen Mischung auf mindestens 8,5 abgesenkt und anschließend durch Zugabe weiterer basischer Lösung auf 7 - 8 eingestellt wird. Der so ausgefällte Feststoff kann anschließend isoliert, getrocknet, zur gewünschten Katalysatorform ausgeformt und darauffolgend kalziniert werden.

### IV. GAS-ZUGABE-VERHÄLTNIS

Im erfindungsgemäßen Verfahren zur Gasphasenhydrierung von Olefinen muß das molare Gas-Zugabe-Verhältnis vorzugsweise so eingestellt werden, daß die Zugabe als Flüssigkeit, vorzugsweise mit einer Temperatur, die bei dem im Gaskreislauf des Reaktorsystems herrschenden Druck unterhalb ihrer Siedetemperatur liegt, in das Gas eingespeist werden kann und im Gas des Gaskreislaufs gasförmig vorliegt. Es ist demgemäß vorzugsweise darauf zu achten, daß der Partialdruck der Zugabe im Gas in einem Bereich liegt, in dem das Gas die Zugabe gasförmig in sich aufnehmen kann, ohne daß eine Vorverdampfung notwendig ist, und Gas und Zugabe im Gaskreislauf vorzugsweise eine homogene Phase bilden. Für den Fall, daß das Gas des erfindungsgemäßen Verfahrens neben Wasserstoff noch Inertgas enthält, ist es bevorzugt, daß die Menge des im Gas enthaltenen Inertgases gleichfalls für die Einstellung des molaren Gas-Zugabe-Verhältnisses berücksichtigt wird. Weiterhin muß in Abhängigkeit der Vollständigkeit der Hydrierung sichergestellt werden, daß mindestens soviel Wasserstoff angeboten wird, daß die gewünschte Zahl an Doppelbindungen hydriert werden kann. Das molare Gas-Zugabe-Verhältnis kann sowohl als molares Wasserstoff-Doppelbindungsverhältnis, als auch - wie in der Großtechnik üblich - als Gasmengen-Zugabegewicht-Verhältnis in Gasmenge bei Normaldruck in Kubikmetern pro kg Zugabe angegeben werden.

Vorzugsweise liegt das molare Gas-Zugabe-Verhältnis im Bereich von 30 bis 150, bevorzugt von 40 bis 130, besonders bevorzugt von 50 bis 100 und darüber hinaus bevorzugt von 60 bis 90.

Vorzugsweise liegt das molare Gas-Zugabe-Verhältnis als molares Wasserstoff-Doppelbindungsverhältnis, insbesondere wenn das Gas mindestens 90 Gew.-% Wasserstoffgas aufweist, im allgemeinen im Bereich von 5 bis 200. In einer bevorzugten Ausführungsform des erfindungsgemäßen Verfahrens liegt das molare Wasserstoff-Doppelbindungsverhältnis im Bereich von 30 bis 100, bevorzugt von 40 bis 130, besonders bevorzugt von 50 bis 100 und darüber hinaus bevorzugt von 60 bis 90.

Darüber hinaus ist es erfindungsgemäß besonders bevorzugt bei einer Zugabe, die mindestens 90 Gew.-% eines C₈-Olefins beinhaltet, ein Gasmengen-Zugabegewicht-Verhältnis, vorzugsweise mit einem Wasserstoffanteil von mindestens 90 Gew.-% am Gas, von 1 bis 30, bevorzugt 5 bis 25 und besonders bevorzugt 10 bis 20 Nm³/kg einzusetzen. Für den Fall, daß die Zugabe mindestens 90 % eines C₁₂-Olefins beinhaltet, ist es erfindungsgemäß besonders bevorzugt, ein Gasmengen-Zugabegewicht-Verhältnis, vorzugsweise mit einem Wasserstoffanteil von mindestens 90 Gew.-% am Gas, von 1 bis 30, bevorzugt 2 bis 20 und besonders bevorzugt 5 bis 15 Nm³/kg im erfindungsgemäßen Verfahren einzustellen.

### V. TEMPERATUR

Im erfindungsgemäßen Verfahren zur katalytischen Hydrierung von Olefinen wird die Temperatur üblicherweise in Abhängigkeit des eingesetzten Katalysators und der eingesetzten Zugabe eingestellt. Hierbei ist es bevorzugt die Temperatur in dem Bereich einzuregeln, in dem der Katalysator aktiv ist. Von diesem Temperaturbereich sind wiederum die im unteren Teil dieses Bereiches liegenden Temperaturen bevorzugt, zumal bei niedriger Temperatur geringere thermische Belastungen des gesamten Reaktionssystems auftreten und die Bildung von bei hohen Temperaturen entstehenden Nebenprodukten unterdrückt wird. Somit erfolgt das erfindungsgemäße Verfahren zur Gasphasenhydrierung und die Einspeisung der Zugabe in das Gas in einem Gastemperaturbereich von 60 bis 200, bevorzugt 70 bis 130 und besonders bevorzugt 70 bis 90 oder 110 bis 130 °C.

### VI. DRUCK

Das erfindungsgemäße Verfahren zur Gasphasenhydrierung von Olefinen wird bevorzugt bei möglichst geringen Drücken durchgeführt, damit es zu keiner hohen Materialbelastung der dem Druck ausgesetzten Bereiche des Reaktionssystems, im folgenden der *Hydrieranlage*, kommt und demgemäß die Hydrieranlage aus weniger druckstabilen Elementen gebildet werden kann, was letztendlich zu einer Kostenreduzierung im Anlagenbau und -betrieb führt. Andererseits ist es je nach Katalysatortyp oftmals notwendig, bei leicht erhöhten Drücken die Hydrierung durchzuführen. Das erfindungsgemäße Verfahren zur Gasphasenhydrierung erfolgt deshalb vorzugsweise in einem Gasdruckbereich von 2 bis 20, bevorzugt 2,5 bis 15 und besonders bevorzugt 3 bis 10 und darüber hinaus bevorzugt 4 bis 7 bar.

### VII. KATALYSATORBELASTUNG

Die Katalysatorbelastung im erfindungsgemäßen Verfahren zur Gasphasenhydrierung von Olefinen ist jeweils von dem eingesetzten Katalysator abhängig. Es ist jedoch bevorzugt, daß sie in einem Bereich von 0,3 bis 2,5, bevorzugt 0,5 bis 2 und besonders bevorzugt von 0,6 bis 1,4 kg/l_{Katalysator}·h liegt.

### VIII. GASKREISLAUF

Im erfindungsgemäßen Verfahren zur Gasphasenhydrierung von Olefinen wird das zuvor beschriebene Gas vorzugsweise in einem Kreislauf geführt. Dieses bedeutet insbesondere, daß das Gas, nachdem es den Reaktor verlassen hat, von den Reaktionsprodukten der Hydrierungsreaktion befreit und der durch die Hydrierungsreaktion verbrauchte Wasserstoff wieder ergänzt wurde, wieder der Hydrierungsreaktion zur Verfügung gestellt wird.

### IX. EINSPEISEN

In dem erfindungsgemäßen Verfahren zur Gasphasenhydrierung von Olefinen kann die Zugabe in jedem Abschnitt des Gaskreislaufs eingespeist werden. Es ist jedoch erfindungsgemäß bevorzugt, daß die Zugabe in dem Abschnitt, der hinter der Abtrennung des Hydrierungsbereichs beginnt und mit dem Reaktor endet, eingespeist sind. Erfindungsgemäß besonders bevorzugt ist es, daß die Zugabe im Reaktor und besonders bevorzugt im oberen Bereich des Reaktors eingespeist wird.

Bevorzugt wird im erfindungsgemäßen Hydrierverfahren die Zugabe mit einer Zugabetemperatur, die bei dem im Gaskreislauf herrschenden Druck unterhalb ihrer Siedetemperatur liegt, in den Gaskreislauf eingespeist, wobei die Zugabe nach der Einspeisung in den Gaskreislauf in diesem vorzugsweise im wesentlichen gasförmig vorliegt. Die Einspeisung der Zugabe erfolgt bevorzugt mit einem höheren als im Gaskreislauf und/oder Reaktor bei der Hydrierung herrschenden Druck. Bevorzugt erfolgt die Einspeisung bei dem 1,01- bis 10-fachen und besonders bevorzugt bei dem 1,5- bis 5-fachen Druck des Gaskreislaufs und/oder Reaktors bei der Hydrierung.

Es ist erfindungsgemäß zudem bevorzugt, daß die Einspeisung durch Eindüsen mittels einer Düse erfolgt. Durch die Verwendung einer Düse ist es möglich, die Zugabe in Form von kleinsten, feinverteilten, Flüssigkeitspartikeln in das Gas des Gaskreislaufs einzuspeisen, so daß es nicht mehr notwendig ist, die Zugabe zur Einspeisung in den Gaskreislauf in einem Verdampfungsbereich zu verdampfen. Die durch die Düse beim Einspeisen der Zugabe in den Gaskreislauf erzeugten Flüssigkeitspartikel sollten vorzugsweise eine mittlere Partikelgröße aufweisen, die es ermöglicht, daß die Zugabe nach der Einspeisung im Gaskreislauf im wesentlichen gasförmig vorliegt. Vor diesem Hintergrund ist eine mittlere Partikelgröße im Bereich von 1 von 100, bevorzugt 1,1 bis 50 und besonders bevorzugt 1,2 bis 20 µm vorteilhaft.

Erfindungsgemäß wird unter dem Begriff "ohne Vorverdampfung auskommen" verstanden, daß der die Zugabe bildende Kohlenwasserstoff und/oder das die Zugabe bildende Kohlenwasserstoffgemisch zwar flüssig in die Hydriervorrichtung eingespeist wird, jedoch ohne Vorverdampfung in dem zuvor definierten Gas gasförmig vorliegt.

### X. HYDRIERANLAGE

Eine bevorzugte Ausführungsform eines Reaktionssystems zur Durchführung des erfindungsgemäßen Gasphasenhydrierverfahrens besteht im Kern aus einem Gaskreislauf (1), in dem das Gas in einer Gasstromrichtung geführt wird, der in Gasstromrichtung aus einem Reaktor, vorzugsweise einem adiabatischen Reaktor, (2), einem Wärmetauscher (3), einem Abscheider (4), einer Frischgaszufuhr (5), einem Kreisgaskompressor (6), einer Zugabedosiereinheit, bestehend aus einem Zugabereservoir (7) und einer Dosierpumpe (8), sowie einem Wärmetauscher (9) besteht. An den Abscheider (4) schließt sich ein weiterer Abscheider (10) zur Trennung von Gas bzw. Wasserstoff (11) von dem entstandenen Hydrierprodukt (12) an. Nach dem Abscheider (4) werden die bei der Reaktion entstehenden Nebenprodukte (13) aus dem Gaskreislauf (1) entfernt. Die einzelnen Baugruppen der zuvor beschriebenen Anlage werden aus dem Anlagenbauer bekannten Bauelementen und Materialien gebildet, die geeignet sind, den Reaktionsbedingungen und insbesondere den Temperatur- und Druckbelastungen des erfindungsgemäßen Verfahrens in geeigneter Weise standzuhalten.

Die Erfindung wird nun anhand von zwei nicht limitierenden Beispielen erläutert.

### BEISPIELE

Die zwei Beispiele wurden gemäß den in den Tabellen 3, 3b, 3c und 3d für Beispiel 1 sowie 4a, 4b und 4c für Beispiel 2 angegebenen Reaktionsparametern durchgeführt, wobei die Reaktion in einem Versuchsreaktor, der in einem Gaskreislauf sich befand durchgeführt wurde. Der Versuchsreaktor bestand aus einem Stahlrohr mit einem Querschnitt von 30 cm und einer Länge von 1 m. Weiterhin wurde er mit einer elektrischen Heizvorrichtung geheizt. In beiden Fällen wurde als Katalysator ein Nickelfällkatalysator in Form von 1,5 mm Strängen mit einer Zusammensetzung von 75 Gew.-% Ni, 15 Gew.-% Si, 5 Gew.-% Al, 5 Gew.-% Zr, jeweils bezogen auf die Oxide, eingesetzt.

### BEISPIEL 1:

**Tabelle 3a**

| **Edukte** | |
|---|---|
| **Zusammensetzung** | **Gew.-%** |
| *Olefine* | |
| 2,4,4-Trimethyl-1-Penten | 69,3 |
| 2,4,4-Trimethyl-2-Penten | 19,3 |
| Sonstige C₈-Olefine | 2,0 |
| C₁₂-Olefine | 7,6 |
| C₁₆-Olefine | 1,0 |

| *Sonstige Verbindungen* | |
|---|---|
| C₄-Kohlenwasserstoffe | 0,6 |
| MTBE | 0,2 |

**Tabelle 3b**

| **Edukteigenschaften** | |
|---|---|
| Bromzahl (g/100 g) | 134,7 |
| Dichte (15 °C) | 0,7241 |
| Brechungsindex | 1,4130 |

**Tabelle 3c**

| **Reaktionsbedingungen** | |
|---|---|
| Druck (bar) | 5 |
| Eintrittstemperatur (°C) | 90 |
| Kohlenwasserstoff-Belastung (kg/(l_{Kat}·h)) | 1 |
| Wasserstoff/Kohlenwasserstoff-Verhältnis (Nm³/kg) | 16 |
| Katalysator | Nickelfällkatalysator nach EP 672 452 |
| Δ T und Reaktor (°C) | 43 |

**Tabelle 3d**

| **Produkteigenschaften** | |
|---|---|
| Bromindex (mg/100 g) | 0 |
| Dichte (15 °C) | 0,7036 |
| Brechungsindex (20 °C) | 1,3961 |

### BEISPIEL 2:

**Tabelle 4a**

| **Edukteigenschaften** Edukt: C₁₂-Olefinschnitt mit 99 Gew.- % Reinheit | |
|---|---|
| Bromzahl (g/100g) | 94,9 |
| Dichte (15 °C) | 0,7733 |
| Brechungsindex (20 °C) | 1,4376 |

**Tabelle 4b:**

| **Reaktionsbedingungen** | |
|---|---|
| Druck (bar) | 5 |
| Eintrittstemperatur | 120 |
| Kohlenwasserstoff-Belastung (kg/(l_{Kat} · h) | 1 |
| Wasserstoff/Kohlenwasserstoff-Verhältnis (Nm³/kg) | 9,5 |
| Katalysator | Nickelfällkatalysator nach EP 672 452 |
| Δ T im Reaktor (°C) | 28 |

**Tabelle 4c:**

| **Produkteigenschaften** | |
|---|---|
| Bromindex (mg/100g) | 0 |
| Dichte (15 °C) | 0,7616 |
| Brechungsindex (20 °C) | 1,4252 |

Die beiden Beispiele zeigen, daß unter Einsatz des erfindungsgemäßen Verfahrens zur Gasphasenhydrierung von Olefinen ein vollständiger Olefinabbau, d.h. eine quantitative Umsetzung des eingesetzten Olefins bei sanften Reaktionsbedingungen erfolgt.

## Patentansprüche

1. Verfahren zur Gasphasenhydrierung von einer mindestens 50 Gew.-% mindestens eines C₆- bis C₂₀-Olefins beinhaltenden Zugabe mit einem mindestens Wasserstoff beinhaltenden Gas an einem Katalysator, wobei die Zugabe als Flüssigkeit in das Gas eingespeist wird.

2. Verfahren nach Anspruch 1, wobei die Gasphasenhydrierung an einem mindestens ein Element der VIII. Nebengruppe beinhaltenden Katalysator in einem Reaktionssystem erfolgt, wobei mit mindestens einem der Parameter
- ein molares Gas-Zugabe-Verhältnis von 5 bis 200,
- ein Gastemperaturbereich von 50 bis 300 °C,
- ein Gasdruckbereich von 1 bis 20 bar,
- eine Katalysatorbelastung in einem Bereich von 0,25 bis 3 kg/l_{Katalysator}·h,
das Gas in einem Gaskreislauf geführt wird.

3. Verfahren nach Anspruch 1 oder 2, wobei die Zugabe mindestens 90 Gew.-% eines C₈- oder C₁₂-Olefins beinhaltet.

4. Verfahren nach einem der vorhergehenden Ansprüche, wobei das Gas Wasserstoff und ein Inertgas, in den Gew.-%-Verhältnissen, jeweils bezogen auf das gesamte Gas, gemäß folgender Tabelle beinhaltet:
| **Gas** | **Gew.-%** | **bevorzugt Gew.-%** | **besonders bevorzugt Gew.-%** |
|---|---|---|---|
| **H**_{**2**}**-Gas** | 50 bis < 100 | 80 bis < 100 | 90 bis < 100 |
| **Inertgas** | >0 bis 50 | >0 bis 20 | >0 bis 10 |

5. Verfahren nach Anspruch 4, wobei als Inertgas Argon und Stickstoff, CH₄ sowie deren Gemische und besonders bevorzugt CH₄ eingesetzt wird.

6. Verfahren nach einem der vorhergehenden Ansprüche, wobei ein Katalysator verwendet wird, der neben mindestens einem Element der VIII. Nebengruppe jeweils mindestens ein Element der III. und IV. Hauptgruppe sowie mindestens ein Element der IV. Nebengruppe aufweist.

7. Verfahren nach Anspruch 6, wobei ein Katalysator verwendet wird, der mindestens die Elemente Ni, Si, Al, Zr aufweist.

8. Verfahren nach einem der vorhergehenden Ansprüche, wobei die Gasphasenhydrierung und die Einspeisung der Zugabe in das Gas bei einem molaren Gas-Zugabe-Verhältnis von 30 bis 150, bevorzugt 40 bis 130, besonders bevorzugt 50 bis 100 und darüber hinaus bevorzugt 60 bis 90 erfolgt.

9. Verfahren nach einem der vorhergehenden Ansprüche, wobei die Gasphasenhydrierung und die Einspeisung der Zugabe in das Gas in einem Gastemperaturbereich von 60 bis 150°C, bevorzugt 70 bis 130°C und besonders bevorzugt 70 bis 100°C oder 110 bis 130°C erfolgt.

10. Verfahren nach einem der vorhergehenden Ansprüche, wobei die Gasphasenhydrierung in einem Druckbereich von 2 bis 20, bevorzugt 2,5 bis 15 und besonders bevorzugt 3 bis 10 bar erfolgt.

## Claims

1. A process for the gas-phase hydrogenation of a feed containing at least 50% by weight of at least one C₆-C₂₀-olefin by means of a gas containing at least hydrogen over a catalyst, wherein the feed is introduced as a liquid into the gas.

2. A process as claimed in claim 1, wherein the gas-phase hydrogenation is carried out over a catalyst containing at least one element of subgroup VIII in a reaction system, the gas being circulated with at least one of the parameters
- a molar gas/feed ratio of from 5 to 200,
- a gas temperature range of from 50 to 300°C,
- a gas pressure range of from 1 to 20 bar,
- a catalyst space velocity of from 0.25 to 3 kg per 1 of catalyst per h.

3. A process as claimed in claim 1 or 2, wherein the feed contains at least 90% by weight of a C₈- or C₁₂-olefin.

4. A process as claimed in any of the preceding claims, wherein the gas contains hydrogen and an inert gas in the weight ratios, based in each case on the total gas, according to the Table below:
| Gas | % by weight | Preferred % by weight | Particularly preferred % by weight |
|---|---|---|---|
| H₂ gas | 50 to < 100 | 80 to < 100 | 90 to < 100 |
| Inert gas | > 0 to 50 | > 0 to 20 | > 0 to 10 |

5. A process as claimed in claim 4, wherein the inert gas used is argon, nitrogen, CH₄ or a mixture thereof, particularly preferably CH₄.

6. A process as claimed in any of the preceding claims, wherein the catalyst used is one which contains at least one element each of main groups III and IV and at least one element of subgroup IV in addition to at least one element of subgroup VIII.

7. A process as claimed in claim 6, wherein the catalyst used contains at least the elements Ni, Si, Al and Zr.

8. A process as claimed in any of the preceding claims, wherein the gas-phase hydrogenation and the introduction of the feed into the gas are carried out at a molar gas/feed ratio of from 30 to 150, preferably from 40 to 130, particularly preferably from 50 to 100, more preferably from 60 to 90.

9. A process as claimed in any of the preceding claims, wherein the gas-phase hydrogenation and the introduction of the feed into the gas are carried out at a gas temperature of from 60 to 150°C, preferably from 70 to 130°C, particularly preferably from 70 to 100°C or from 110 to 130°C.

10. A process as claimed in any of the preceding claims, wherein the gas-phase hydrogenation is carried out at from 2 to 20, preferably from 2.5 to 15, particularly preferably from 3 to 10, bar.

## Revendications

1. Procédé d'hydrogénation en phase gazeuse d'une charge contenant au moins 50% en poids d'au moins une oléfine en C₆ à C₂₀ au moyen d'un gaz contenant au moins de l'hydrogène, sur un catalyseur, la charge étant amenée sous forme liquide dans le gaz.

2. Procédé selon la revendication 1, où l'hydrogénation en phase gazeuse est entreprise sur un catalyseur contenant au moins un élément du VIIIème sous-groupe dans un système réactionnel, où le gaz est envoyé dans un circuit de gaz avec au moins l'un des paramètres
- une proportion molaire gaz - charge de 5 à 200,
- une plage de température de gaz de 50 à 300°C,
- une plage de pression de gaz de 1 à 20 bar,
- une charge de catalyseur de l'ordre de 0,25 à 3 kg/l _{catalyseur}. h.

3. Procédé selon la revendication 1 ou 2, où la charge contient au moins 90% en poids d'une oléfine en C₈ ou C₁₂.

4. Procédé selon l'une des revendications qui précèdent, où le gaz contient de l'hydrogène et un gaz inerte, dans les proportions de pourcentages en poids, à chaque fois par rapport à la totalité du gaz, selon le tableau suivant:
| Gaz | % en poids | % en poids préférentiels | % en poids particulièrement préférentiels |
|---|---|---|---|
| H₂ gazeux | 50 à <100 | 80 à <100 | 90 à <100 |
| Gaz inerte | >0 à 50 | >0 à 20 | >0 à 10 |

5. Procédé selon la revendication 4, où l'on met en oeuvre comme gaz inerte de l'argon et de l'azote, CH₄, ou leurs mélanges, et de manière particulièrement préférentielle du CH₄.

6. Procédé selon l'une des revendications qui précèdent, où l'on utilise un catalyseur qui, outre au moins un élément du VIIIème sous-groupe, présente à chaque fois au moins un élément des IIIème et IVème sous-groupes ainsi qu'au moins un élément du IVème sous-groupe.

7. Procédé selon la revendication 6, où l'on utilise un catalyseur qui présente au moins les éléments Ni, Si, Al, Zr.

8. Procédé selon l'une des revendications qui précèdent, où l'on entreprend l'hydrogénation en phase gazeuse et l'amenée de la charge dans le gaz avec une proportion molaire gaz - charge de 30 à 150, de préférence de 40 à 130, plus préférentiellement de 50 à 100 et de manière encore plus préférentielle de 60 à 90.

9. Procédé selon l'une des revendications qui précèdent, où l'on entreprend l'hydrogénation en phase gazeuse et l'amenée de la charge dans le gaz dans une plage de température de gaz de 60 à 150°c, de préférence de 70 à 130°C et de manière particulièrement préférentielle de 70 à 110°C ou de 110 à 130°C.

10. Procédé selon l'une des revendications qui précèdent, où l'on entreprend l'hydrogénation en phase gazeuse dans une plage de pression de 2 à 20, de préférence de 2,5 à 15 et de manière particulièrement préférentielle de 3 à 10 bar.
